# EUROPEAN PATENT APPLICATION

(11) **EP 4 621 054 A1**
(43) Date of publication of application: **24.09.2025**
(21) Application number: 23891568.0
(22) Date of filing: 14.11.2023
(51) Int. Cl.: C12N 15/10, C12Q 1/6806

(54) **METHOD FOR COLLECTING MIRNA, COLLECTION MEMBRANE FILTER, COLLECTION MEMBRANE FILTER UNIT, AND COLLECTION KIT**

(30) Priority: 15.11.2022 JP 2022182299
(71) Applicant: Kansai Medical University Educational Corporation, City of Hirakata, Osaka 573-1010 (JP)
(72) Inventor: KANDA, Seiji, Hirakata-shi, Osaka 573-1010 (JP)
(74) Representative: Marks & Clerk LLP
(86) International application number: PCT/JP2023/040901
(87) International publication number: WO 2024/106421

(57) **Abstract**

The present invention solves one problem of providing a method for collecting miRNA in high yield. The problem is solved by a method for collecting miRNA from a liquid specimen collected from a subject, said method comprising contacting the liquid specimen with capture probe-bound cationic polymer-modified PLGA nanoparticles and recovering the miRNA from the capture probe-bound cationic polymer-modified PLGA nanoparticles contacted with the liquid specimen, wherein the capture probe-bound cationic polymer-modified PLGA nanoparticle comprises a core of the nanoparticle containing PLGA, a coating layer containing a cationic polymer covering the surface of the core, and a capture probe bound to the coating layer, the capture probe being an oligonucleotide.

## Description

### Technical Field

Disclosed herein are a method for collecting miRNA, a collection membrane filter, a collection membrane filter unit, and an enrichment kit.

### Background Art

Patent Literature 1 discloses a method for producing a poly (lactic-co-glycolic acid) copolymer (PLGA) nanoparticle including lipophilic ascorbyl tetrahexyldecanoate.

Patent Literature 2 discloses a method for producing a PLGA nanoparticle with a particle size that can pass through a membrane filter for filtration sterilization.

Patent Literature 3 discloses a PLGA nanoparticle including an asORN corresponding to a specific region of antisense RNA for interferon-α.

Non Patent Literature 1 describes a method for extracting DNA and RNA using Urine Conditioning Buffer^{™}.

### Citation List

### Patent Literature

[Patent Literature 1] Japanese Unexamined Patent Application No. 2005-213170
[Patent Literature 2] Japanese Unexamined Patent Application No. 2011-111429
[Patent Literature 3] Japanese Unexamined Patent Application No. 2016-130227

### Non Patent Literature

[Non Patent Literature 1] Set of reagents and containers for preservation of DNA and RNA in urine samples at room temperature| Urine Collection Kit with Urine Conditioning Buffer (UCB) | Funakoshi Co., Ltd. (funakoshi.co.jp) Date: 2017/05/29

### Summary of Invention

### Technical Problem

As a non-invasive urinary biomarker for early diagnosis of cancer and tuberculosis, collection and analysis of miRNA in urine and other liquid specimens collected from a subject are performed.

However, since the amount of the miRNA present in the liquid specimen is very low, it is necessary to collect the miRNA in high yield. In addition, it is difficult to selectively collect specific miRNA by the method described in Non Patent Literature 1.

The present invention solves one problem of providing a method for collecting the miRNA in high yield.

### Solution to Problem

Item 1. A method for collecting miRNA from a liquid specimen collected from a subject comprising;
contacting the liquid specimen with capture probe-bound cationic polymer-modified PLGA nanoparticles; and
recovering the miRNA from the capture probe-bound cationic polymer-modified PLGA nanoparticles contacted with the liquid specimen, wherein
the capture probe-bound cationic polymer-modified PLGA nanoparticle comprises
   a core of the nanoparticle containing PLGA, a coating layer containing a cationic polymer covering the surface of the core, and a capture probe bound to the coating layer, the capture probe being an oligonucleotide.

Item 2. The method according to item 1, wherein the cationic polymer is chitosan.

Item 3. The method according to item 1, wherein the oligonucleotide contains a random sequence or a target miRNA-specific sequence.

Item 4. A collection membrane filter for collecting miRNA from a liquid specimen,
the collection membrane filter being supported with capture probe-bound cationic polymer-modified PLGA nanoparticles, wherein the capture probe-bound cationic polymer-modified PLGA nanoparticle comprises a core of the nanoparticle containing the PLGA, a coating layer containing a cationic polymer covering the surface of the core, and a capture probe bound to the coating layer, the capture probe being an oligonucleotide.

Item 5. A collection membrane filter unit for collecting miRNA from a liquid specimen, having a filter folder incorporating the collection membrane filter according to item 4, an injection tube, and a discharge tube.

Item 6. A kit for collecting miRNA from a liquid specimen comprising the collection membrane filter unit according to item 5 and an injector for injecting the liquid specimen into the collection membrane filter unit.

### Advantageous Effects of Invention

Collection of the miRNA, which are present only in very small amounts in the specimen, can be achieved.

### Brief Description of Drawings

FIG. 1 shows an overview of a method for preparing a capture probe-bound cationic polymer-modified PLGA nanoparticle.
FIG. 2A shows an external view of a collection membrane filter unit 1 for collecting miRNA. FIG. 2B shows a median cross-sectional view of the collection membrane filter unit 1 for collecting the miRNA.
FIG. 3 shows an overview of a method for collecting miRNA in a liquid specimen using the capture probe-bound cationic polymer-modified PLGA nanoparticle. FIG. 3A shows an overview of a first embodiment. FIG. 3B shows an overview of a second embodiment.
FIG. 4 shows an amount of small RNA and miRNA in urine recovered. FIG. 4A shows an amount of small RNA and miRNA recovered in a control in which the PLGA nanoparticles were not added. FIG. 4B shows an amount of small RNA and miRNA recovered directly from the urine without using chitosan-modified PLGA nanoparticles to which capture probes were not bound. FIG. 4C shows an amount of small RNA and miRNA recovered using Urine Conditioning Buffer^{™}. FIG. 4D shows an amount of small RNA and miRNA recovered directly from the urine using capture probe-bound chitosan-modified PLGA nanoparticles.
FIG. 5A is an image of a nitrocellulose filter supported with chitosan-modified PLGA nanoparticles by scanning electron microscopy. FIG. 5B is an image of a PVDF filter supported with the chitosan-modified PLGA nanoparticles by the scanning electron microscopy.

### Description of Embodiments

### 1. Capture probe-bound cationic polymer-modified PLGA nanoparticle

One embodiment relates to a cationic polymer-modified PLGA (poly (lactic-co-glycolic acid)) nanoparticle to which a capture probe is bound.

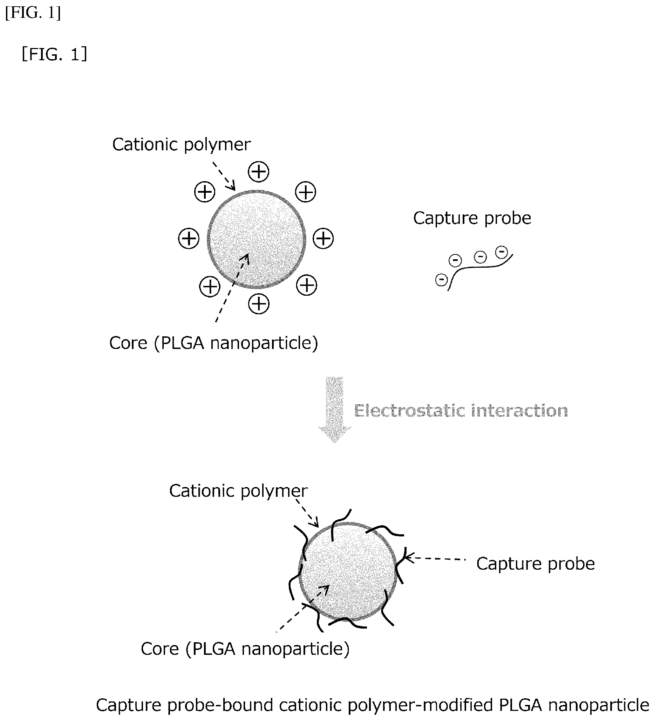

FIG. 1 shows a schematic diagram of a preparation method for the capture probe-bound cationic polymer-modified PLGA nanoparticle and a schematic diagram of the prepared capture probe-bound cationic polymer-modified PLGA nanoparticle.

A core of the cationic polymer-modified PLGA nanoparticle is a nanoparticle containing PLGA. The surface of the core is provided with a coating layer containing a cationic polymer.

A molecular weight of the PLGA used as the core is preferably in a range of 5,000 to 200,000, and more preferably in a range of 15,000 to 25,000. A composition ratio of lactic acid to glycolic acid may be 1:99 to 99:1, but 1:0.333 is preferred.

Preferred examples of the cationic polymer include, chitosan and chitosan derivatives, cationized cellulose formed by bonding a plurality of cationic groups to cellulose, polyamino compounds such as polyethylenimine, polyvinylamine, and polyallylamine, polyamino acids such as polyornithine and polylysine, polyvinylimidazole, polyvinylpyridinium chloride, alkylaminomethacrylate quaternary salt polymer (DAM), alkylaminomethacrylate quaternary salt-acrylamide copolymer (DAA), cationic polymer (e.g., copolymer of MPC and (2-hydroxy-3-methacryloyloxypropyl) trimethylammonium chloride) in which a cationic group such as a quaternary ammonium salt is bonded to a polymer whose constituent unit is 2-methacryloyloxyethyl phosphorylcholine (MPC) having both a phospholipid polar group (phosphorylcholine group) which is a component of cell membrane (biomembrane) and a methacryloyl group with excellent polymerizability, and the like. Particularly, the chitosan or its derivative is suitably used. As an example of the chitosan derivative, hydroxypropyl chitosan (cationized chitosan) can be included. An amount of the cationic polymer coating is, without being limited, usually about from 1 to 10 parts by weight, preferably about from 3 to 8 parts by weight, based on 100 parts by weight of the PLGA.

### (Nanoparticle formation step)

A PLGA nanoparticle is produced by electrospray deposition (ESD) method. The ESD method is a method in which an emulsion is formed and then a polymer is crystallized into a spherical shape by using interdiffusion between a good solvent (organic solvent) and a poor solvent (hydrophilic solvent containing the cationic polymer and polyvinyl alcohol). As an operation procedure, the PLGA is first dissolved in the good solvent and dropped into the poor solvent under stirring, whereby the good solvent in the mixture is diffusely transferred into the poor solvent rapidly. As a result, self-emulsification of the good solvent occurs in the poor solvent, and a submicron-sized emulsion drop of the good solvent is formed. In addition, as the interdiffusion between the good and poor solvents proceeds, the solubility of the PLGA in the emulsion drop decreases and the PLGA nanoparticle in the form of spherical crystalline particle is finally formed. Furthermore, by adding polyvinyl alcohol and the cationic polymer to the poor solvent, the surface of the PLGA nanoparticle is coated with the polyvinyl alcohol and the cationic polymer, and the cationic polymer-modified PLGA nanoparticle is formed. That is, the PLGA nanoparticle comprises a structure in which the PLGA is the core, the polyvinyl alcohol coats the core, and the cationic polymer coats an outer layer of the core.

A concentration of the PLGA in the good solvent is about 8 mg/mL to 15 mg/mL, preferably about 10 mg/mL to 13 mg/mL. The good solvent preferably contains acetone and ethanol. The mixing ratio of the acetone and ethanol in the good solvent is not particularly limited, but it is preferable that an ethanol concentration is 10 vol% or more. If necessary, a solvent other than the acetone and ethanol such as water may be mixed in the good solvent.

As the poor solvent, water can be used. A concentration of the polyvinyl alcohol in the poor solvent can range from 0.05 wt% to 10 wt%.

### (Distilling away step)

After the cationic polymer-modified PLGA nanoparticle is formed, the organic solvent, which is the good solvent, is distilled away by evaporation under reduced pressure to obtain a nanoparticle suspension. In this distilling away step, if the organic solvent, which is the good solvent, is distilled away by evaporation under reduced pressure for a long time at a temperature exceeding a glass transition temperature of a biocompatible polymer, rigidity of the biocompatible polymer composing the nanoparticle decreases and fluidity increases, causing the nanoparticles to fuse with each other, and pressure filtration performance is significantly reduced in a filter sterilization step following the distilling away step. Therefore, the distilling away step should be performed at a temperature as low as possible and in a time as short as possible. Specifically, the step is preferably performed at 45°C or lower for 30 hours or less.

A particle size of the thus prepared capture probe-bound cationic polymer-modified PLGA nanoparticles averages about 240 nm.

### (Binding of capture probe)

A capture probe binds to the coating layer of the cationic polymer-modified PLGA nanoparticle. The capture probe contains an oligonucleotide. The oligonucleotide contains a random sequence, such as a random primer, or a target miRNA-specific sequence. The oligonucleotide is about 6 to 20 nucleotides in length.

As the random primer, for example, a random primer (hexamer) from Promega Corporation can be used.

The target miRNA-specific sequence can include a sequence complementary to miR451a, etc. for active tuberculosis infection, or miR-192, miR-18a, miR-221, etc. for pancreatic cancer. Use of the target miRNA-specific sequence can increase an amount of specific miRNA recovered, unlike a recovery method described in Non-Patent Literature 1.

The capture probe is bound to the coating layer of the cationic polymer-modified PLGA nanoparticle via electrostatic binding. Since the surface of the cationic polymer-modified PLGA nanoparticle is positively charged, a negatively charged substance such as a nucleic acid can be bound electrostatically to the cationic polymer-modified PLGA nanoparticle.

Specifically, oligonucleotides and, for example, mannitol can be added to the suspension of the cationic polymer-modified PLGA nanoparticles, and the suspension is freeze-dried and powdered at, for example, -45°C to obtain the capture probe-bound cationic polymer-modified PLGA nanoparticles.

### 2. Collection membrane filter for collecting miRNA

One embodiment relates to a collection membrane filter for collecting the miRNA (hereinafter also referred to simply as "collection membrane filter").

The membrane filter is a membrane having pores. A pore diameter is preferably about 0.1 µm to 0.45 µm. In addition, a material of the membrane filter can include, without being particularly limited, nitrocellulose, glass, polyethersulfone, polycarbonate, cellulose acetate, polytetrafluoroethylene (PTFE), polyvinylidene difluoride (PVDF), and the like. An organic solvent-resistant material such as the PVDF is preferred.

The capture probe-bound cationic polymer-modified PLGA nanoparticles can be supported on the membrane filter by the following method.

For example, a suspension containing about 0.01 to 20 mg/ml of the capture probe-bound cationic polymer-modified PLGA nanoparticles is prepared. A solvent for preparing the said suspension is preferably a hydrophilic solvent such as water or buffer. The membrane filter is set in a suction filtration device. When the membrane filter is a hydrophobic PVDF membrane or the like, it is preferable that the membrane filter is first infiltrated with methanol, followed by swelling with the hydrophilic solvent. The said suspension can be applied onto the membrane filter and sucked, whereby the capture probe-bound cationic polymer-modified PLGA nanoparticles are supported on the membrane filter, and the hydrophilic solvent is removed to prepare the collection membrane filter.

### 3. Collection membrane filter unit for collecting miRNA

One embodiment relates to a collection membrane filter unit 1 for collecting the miRNA (hereinafter also referred to simply as "filter unit 1").

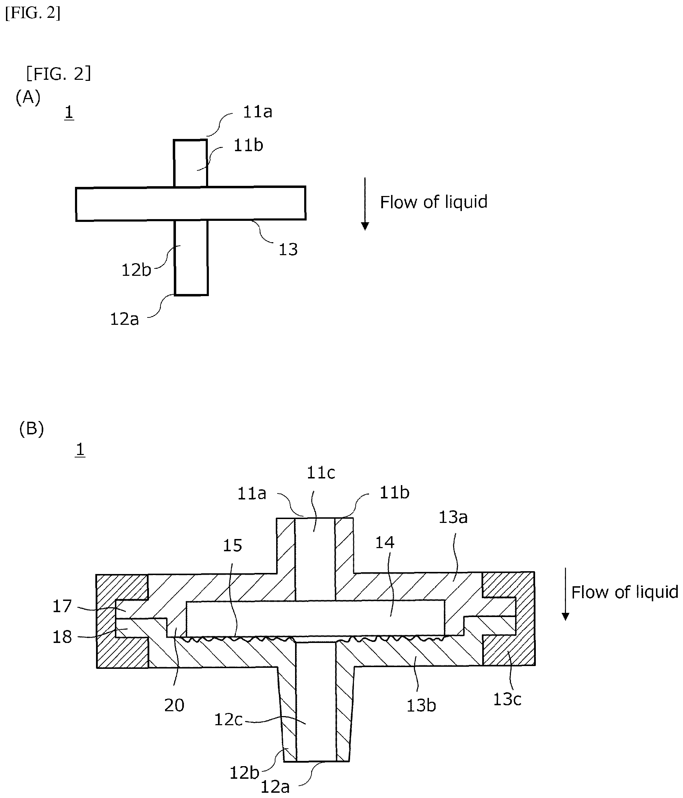

A structure of the filter unit 1 will be described with reference to FIGS. 2A and 2B. The filter unit 1 can have a structure described in, for example, JPS5531480A and others. The filter unit 1 has a filter folder 13 incorporating the collection membrane filter 15 prepared in the above section 2, an injection tube 11b, and a discharge tube 12b. In FIG. 2A, a code 11a indicates an injection port and a code 12a represents a discharge port. The injection port 11a is connected with an injector (for example, an injection syringe) for injecting a liquid specimen. The connection is made by inserting a tip on a discharge side of the injector (in case of the injection syringe, the tip to which an injection needle is usually attached) into the injection port 11a of the filter unit 1. In addition, in order to prevent the filter unit 1 from coming off under pressure when injecting the liquid specimen, the injection port 11a of the filter unit 1 preferably has a female luer lock structure. The filter folder 13 is preferably made of plastic so as to be disposable.

A flow of injected liquid specimen is indicated by an arrow in FIG. 2A.

Next, a structure inside the filter folder13 will be described with reference to FIG. 2B. FIG. 2B is a vertical cross-sectional view of the filter unit 1 at the midline.

The filter folder 13 is disk-shaped and has a two-layer structure of a filter folder upper portion 13a and a filter folder lower portion 13b, and a tightening ring 13c for sealing the collection membrane filter 15, the filter folder upper portion 13a, and the filter folder lower portion 13b. The injection tube 11b is connected to the center of the filter folder upper portion 13a, and the inside of the injection tube 11b is a luminal structure, which is an injection lumen 11c. The discharge tube 12b is connected to the center of the filter folder lower portion 13b, and the inside of the discharge tube 12b is a luminal structure, which is a discharge lumen 12c. The filter folder upper portion 13a and the filter folder lower portion 13b are designed so that when the collection membrane filter 15 is sandwiched therebetween and tightened by the tightening ring 13c, a disk-shaped space 14 is formed on the filter folder upper portion 13a side. This space 14 is filled with liquid when the liquid is injected and serves as a pressure chamber. The space 14 is formed by an inside of a collar 20, which is extended in a circular manner below the filter folder upper part 13a. The top of the filter folder lower portion 13b is formed into a concave structure so as to be fitted to an outer edge of the collar 20. The bottom of the concave structure has a groove cut in the shape of a spider web from an opening in the discharge tube 12b, which is designed to provide a flow path for the liquid that has passed through the collection membrane filter 15.

An annular outward flange 17 is formed on the lower lateral side of the filter folder upper portion 13a. In addition, an annular outward flange 18 complementary to the flange 17 is formed on the upper lateral side of the filter folder lower portion 13b. It is designed so that when the filter folder upper portion 13a and the filter folder lower portion 13b are overlapped, the tightening ring 13c is fitted to the portion where the flange 17 and the flange 18 are overlapped.

The collection membrane filter 15 is circular in shape and is positioned so that it does not sit between the flanges 17 and 18, but it overlies the fitted portion of the collar 20 and the concave structure of the filter folder lower portion 13b, and is sandwiched and fixed between the collar 20 and the bottom of the concave structure of the filter folder lower portion 13b.

The liquid injected from the injection port 11a is filled into the space 14 through the injection tube 11b, passed through the collection membrane filter 15 by the pressure of the injector into which the liquid is injected, and discharged from the discharge port 12a through the discharge tube 12b.

As the liquid specimen passes through the collection membrane filter 15, the miRNA in the liquid specimen is collected by the capture probe-bound cationic polymer-modified PLGA nanoparticles.

The tightening ring 13c is removable, and the collection membrane filter 15 through which the liquid specimen is filtered can be collected by removing the tightening ring 13c.

The size of the collection membrane filter 15 is about 10 mm to 35 mm in diameter. Accordingly, a diameter of the filter folder 13 is also about 13 mm to 40 mm. If a height is defined as the height from the injection port 11a to the discharge port 12a, the height is about 20 mm to 30 mm.

### 4. Kit for collecting miRNA from liquid specimen

One embodiment relates to a kit for collecting the miRNA from the liquid specimen.

The kit includes the filter unit 1 and the injector described in the above section 3. The injector can be an injection syringe or the like. It is preferable that the injector can contain at least 10 mL of the liquid specimen.

### 5. Collection of miRNA in liquid specimen using capture probe-bound cationic polymer-modified PLGA nanoparticle

One embodiment relates to a method for collecting the miRNA in the liquid specimen (hereinafter sometimes simply referred to as "collection method").

The collection method comprises contacting the liquid specimen with the capture probe-bound cationic polymer-modified PLGA nanoparticle and recovering the miRNA from the capture probe-bound cationic polymer-modified PLGA nanoparticle contacted with the liquid specimen.

The liquid specimen can be urine, serum, plasma, pleural effusion, ascites, spinal fluid, joint fluid, puncture fluid, puncture fluid other than the above, throat swab, and the like which may be collected from the subject. The urine is preferred. For highly viscous throat swabs and the like, a cotton swab wiped on a throat may be washed in sterile saline solution and the wash solution may be used as the specimen.

The subject can include human, dog, cat, rabbit, mouse, rat, monkey, cow, horse, sheep, goat or the like.

The collection method can include the following two embodiments.

### (1) First embodiment

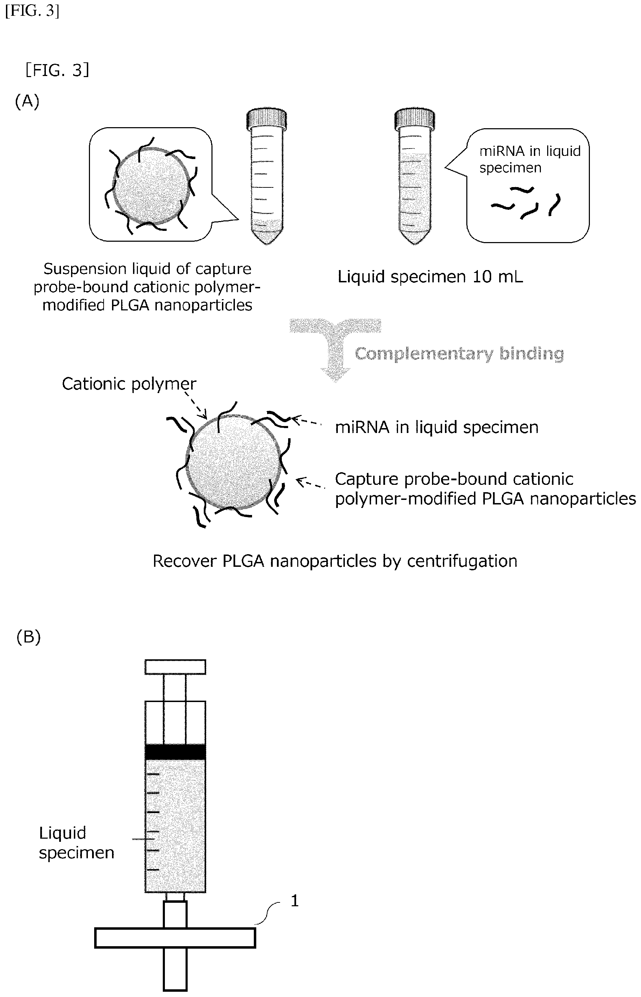

A first embodiment is a method in which the liquid specimen is contacted with the capture probe-bound cationic polymer-modified PLGA nanoparticles by directly adding the capture probe-bound cationic polymer-modified PLGA nanoparticles described in the above section 1 to the liquid specimen, and the capture probe-bound cationic polymer-modified PLGA nanoparticles after the contact are collected. An overview of the collection method is shown in FIG. 3A. If miRNA that hybridizes to the capture probe is present in the liquid specimen, it is collected by the capture probe.

The capture probe-bound cationic polymer-modified PLGA nanoparticles can be recovered, for example, by precipitating the capture probe-bound cationic polymer-modified PLGA nanoparticles after contact with the liquid specimen by centrifugation at about 40,000 to 50,000 × g for about 20 to 40 min at 0 to 10°C, and then removing the supernatant.

### (2) Second embodiment

A second embodiment is the method using the filter unit 1 described in the above section 3. An overview is shown in FIG. 3B. The liquid specimen is set in the injector and the filter unit 1 is set at the tip of the injector.

The liquid specimen is pushed out of the injector and a filtrate passed through the collection membrane filter 15 is discarded from the discharge port 12a of the filter unit 1. The liquid specimen comes into contact with the capture probe-bound cationic polymer-modified PLGA nanoparticles supported on the collection membrane filter 15, and if miRNA that hybridize to the capture probe is present in the liquid specimen, it is collected by the capture probe.

The tightening ring 13c is removed to retrieve the collection membrane filter 15.

### 6. Recovery of miRNA from capture probe-bound cationic polymer-modified PLGA nanoparticle

In the first embodiment described in the above section 5, the recovery of the miRNA from the capture probe-bound cationic polymer-modified PLGA nanoparticles contacted with the liquid specimen can be achieved by adding a phenol/chloroform mixture to the capture probe-bound cationic polymer-modified PLGA nanoparticles, performing a phenol/chloroform extraction, and then performing ethanol precipitation.

In the second embodiment described in the above section 5, the retrieved collection membrane filter 15 is immersed in acetone or toluene to dissolve the PLGA nanoparticles and elute nucleic acids from the membrane filter. The miRNA can be recovered by adding the phenol/chloroform mixture to the eluate, performing the phenol/chloroform extraction, and then performing the ethanol precipitation.

### Examples

Hereinafter, the present invention will be described in more detail with reference to examples. However, the present invention should not be construed as limited to the examples.

### 1. Preparation of capture probe-bound chitosan-modified PLGA nanoparticle

A chitosan-modified PLGA nanoparticle was prepared by ESD method using PLGA base material (PLGA-7520; Polymerization ratio of lactic acid to glycolic acid = 3:1, average molecular weight of 20,000 Da) as follows.

200 mg of PLGA was dissolved in 13 mL of acetone, which is a good solvent for the PLGA, to obtain a polymer solution. Then, 4 mL of ethanol was added to the solution and mixed to obtain a mixed good solvent. Next, 525 mg of 2 wt% chitosan (KIMICA Chitosan, manufactured by KIMICA Corporation) aqueous solution was added to 5 g of 2 wt% polyvinyl alcohol (PVA: GOHSENOL EG-05, manufactured by The Nippon Synthetic Chemical Industry Co., Ltd.) aqueous solution, and mixed to obtain a poor solvent for the PLGA. The mixed good solvent was dropped into this poor solvent at a constant rate (20 mL/min) under stirring at 400 rpm at 40°C to obtain a suspension of PLGA nanoparticles by diffusion phenomenon of the good solvent into the poor solvent. Subsequently, the acetone and ethanol were removed under reduced pressure, and a random primer (Promega Corporation: Hexamer) was then added to the obtained suspension of the nanoparticles at a final concentration of 0.0056%, and the suspension was freeze-dried and powdered at -45°C to obtain a random primer-bound chitosan-modified PLGA nanoparticle powder.

### 2. Recovery of small RNA and miRNA in urine using random primer-bound chitosan-modified PLGA nanoparticle

About 2 ml of purified water was added to 10 mg of the powdered random primer-bound chitosan-modified PLGA nanoparticles (hereinafter also referred to simply as "PLGA nanoparticles") and sonicated to disperse homogeneously.

2 ml of the suspension of the PLGA nanoparticles was added to 10 mL of urine, and mixed them.

Thereafter, the PLGA nanoparticles were precipitated by centrifugation at 48,000 × g at 4°C for 30 min and the supernatant was removed.

A mixture of QIAzol (from QIAGEN) and chloroform (5:1) was added to the precipitate, and phenol-chloroform extraction and ethanol precipitation were performed to recover the RNA in the urine.

### 3. Amount of small RNA and miRNA recovered

An amount of the RNA recovered was determined by capillary electrophoresis. The 2100 Bioanalyzer electrophoresis system from Agilent Technologies, Inc. was used for the capillary electrophoresis, and the amount of the RNA was quantified using the 2100 Expert Software.

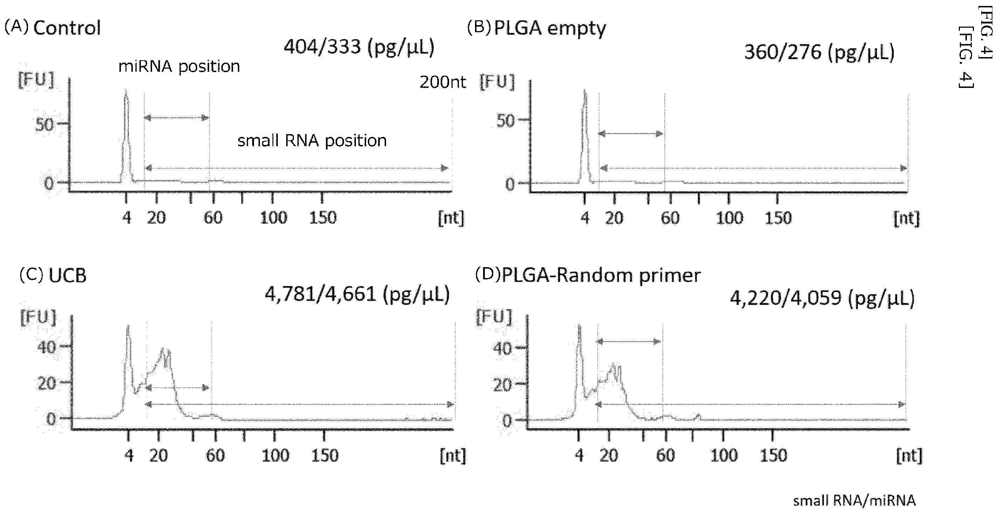

FIG. 4 shows an amount of small RNA and miRNA in urine recovered. FIG. 4A shows an amount of small RNA and miRNA recovered in a control in which the PLGA was not added. FIG. 4B shows an amount of small RNA and miRNA recovered directly from the urine without using the chitosan-modified PLGA nanoparticles to which capture probes were not bound. FIG. 4C shows an amount of small RNA and miRNA recovered using Urine Conditioning Buffer^{™} (UCB). FIG. 4D shows an amount of small RNA and miRNA recovered directly from the urine using the capture probe-bound chitosan-modified PLGA nanoparticles.

The amount of the RNA recovered, shown as small RNA/miRNA (pg/µL), was 404/333 for the control, 360/276 for PLGA empty, 4,781/4,661 for the UCB, and 4,220/4,059 for the capture probe-bound chitosan-modified PLGA nanoparticle (PLGA-Random primer). The amount of the small RNA and the miRNA recovered using the capture probe-bound chitosan-modified PLGA nanoparticles was similar to that using the UCB.

### 4. Supporting test of chitosan-modified PLGA nanoparticle on filter

Chitosan-modified PLGA nanoparticles were supported onto a nitrocellulose filter [HABG (CAT. NO. HABG04700)] with a pore size of 0.45 µm and a PVDF filter (Durapore GV) with a pore size of 0.22 µm using a suction filtration device. Each of the filters was placed in a filter installation portion of the suction filtration device, and water was first placed in an upper chamber of the suction filtration device and sucked using water flow, and then the water was dropped into a lower chamber of the suction filtration device. Next, a suspension of the chitosan-modified PLGA nanoparticles (2.2752 g of the suspension contains 22.4 mg of the chitosan-modified PLGA nanoparticles) was placed in the upper chamber of the suction filtration device and sucked using water flow. Water was again placed in the upper chamber of the suction filtration device and sucked using water flow. The filters after the filtration were retrieved and frozen. The frozen filters were fixed, dehydrated, and evaporated, and the surfaces of the filters were observed using a scanning electron microscopy.

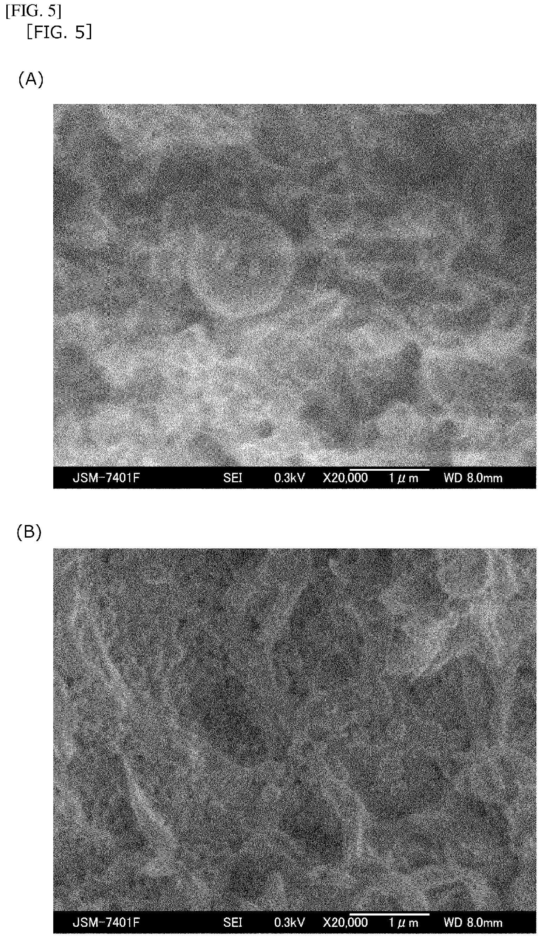

Results are shown in FIG. 5. FIG. 5A is an image of the nitrocellulose filter supported with the chitosan-modified PLGA nanoparticles by the scanning electron microscopy. FIG. 5B is an image of the PVDF filter supported with the chitosan-modified PLGA nanoparticles by the scanning electron microscopy. In both cases, approximately spherical chitosan-modified PLGA nanoparticles were supported on the filter.

## Claims

1. A method for collecting miRNA from a liquid specimen collected from a subject comprising;
contacting the liquid specimen with a capture probe-bound cationic polymer-modified PLGA nanoparticle; and
recovering the miRNA from the capture probe-bound cationic polymer-modified PLGA nanoparticle contacted with the liquid specimen, wherein
the capture probe-bound cationic polymer-modified PLGA nanoparticle comprises
a core of the nanoparticle containing PLGA, a coating layer containing a cationic polymer covering the surface of the core, and a capture probe bound to the coating layer, the capture probe being an oligonucleotide.

2. The method according to claim 1, wherein the cationic polymer is chitosan.

3. The method according to claim 1, wherein the oligonucleotide contains a random sequence or a target miRNA-specific sequence.

4. A collection membrane filter for collecting miRNA from a liquid specimen,
the collection membrane filter being supported with a capture probe-bound cationic polymer-modified PLGA nanoparticle, wherein
the capture probe-bound cationic polymer-modified PLGA nanoparticle comprises
a core of the nanoparticle containing PLGA, a coating layer containing a cationic polymer covering the surface of the core, and a capture probe bound to the coating layer, the capture probe being an oligonucleotide.

5. A collection membrane filter unit for collecting miRNA from a liquid specimen, having a filter folder incorporating the collection membrane filter according to claim 4,
an injection tube, and
a discharge tube.

6. A kit for collecting miRNA from a liquid specimen comprising
the collection membrane filter unit according to claim 5 and
an injector for injecting the liquid specimen into the collection membrane filter unit.
